# EUROPEAN PATENT SPECIFICATION

(11) **EP 1 255 754 B1**
(45) Date of publication and mention of the grant of the patent: **15.06.2005**
(21) Application number: 01904805.7
(22) Date of filing: 29.01.2001
(51) Int. Cl.: C07D 413/10

(54) **LINEZOLID-CRYSTAL FORM II**
LINEZOLID-KRYSTALL FORM II
FACIES DE CRISTAUX DE LINEZOLIDE (FORME II)

(30) Priority: 02.02.2000 US 179837 P
(43) Date of publication of application: 13.11.2002
(73) Proprietor: Pharmacia & Upjohn Company LLC, Kalamazoo, MI 49001 (US)
(72) Inventor: BERGREN, Michael, S., Portage, MI 49002 (US)
(74) Representative: Perry, Robert Edward
(86) International application number: PCT/US2001/000657
(87) International publication number: WO 2001/057035

(56) References cited:
- WO-A-99/24393
- US-A- 5 688 792
- US-A- 5 837 870
- "LINEZOLID. OXAZOLIDINONE ANTIBACTERIAL" DRUGS OF THE FUTURE,ES,BARCELONA, vol. 21, no. 11, 1996, pages 1116-1123, XP000654643 ISSN: 0377-8282
- ZURENKO G E ET AL: "OXAZOLIDINONE ANTIBACTERIAL AGENTS: DEVELOPMENT OF THE CLINICAL OXAZOLIDINONE ANTIBACTERIAL AGENTS: DEVELOPMENT OF THE CLINICAL CANDIDATES EPEREZOLID AND LINEZOLID. CANDIDATES EPEREZOLID AND LINEZOLID" EXPERT OPINION ON INVESTIGATIONAL DRUGS,GB,ASHLEY PUBLICATIONS LTD., LONDON, vol. 6, no. 2, 1997, pages 151-158, XP000654528 ISSN: 1354-3784
- J. MED. CHEM., vol. 39, 1996, pages 673-679, XP002168553 cited in the application

## Description

### BACKGROUND OF THE INVENTION

### 1. Field of the Invention

The field of the invention is a novel crystal form of a known compound, linezolid which is pharmaceutically useful as an antibacterial agent.

### 2. Description of the Related Art

US Patent 5,688,792 discloses the antibacterial agent linezolid as well as a process for its preparation. EXAMPLE 5 reports the linezolid produced had a mp of 181.5-182.5°.

There are many other disclosures of processes to prepare linezolid. *J. Med. Chem.,* 39(3), 673-9 (1996) reports the linezolid was, "recrystallized from ethyl acetate and hexanes ...white crystals, m.p. 181.5-182.5C." It also sets forth the IR spectrum as "3284, 3092, 1753, 1728, 1649, 1565, 1519, 1447, 1435".

*Tetrahedron Lett*., 40(26), 4855 (1999) discloses linezolid and a process to prepare linezolid. However, this document does not set forth the melting point or IR spectrum of the linezolid prepared.

US Patent 5,837,870 (International Publication WO97/37980 of PCT/US97/03458) discloses a process to prepare linezolid. Linezolid is described in EXAMPLE 18, which does not set forth the melting point or IR spectrum of the linezolid prepared.

International Publication WO99/24393 of PCT/US98/20934 discloses a process to prepare linezolid. Linezolid is described in EXAMPLES 8, 9 and 12 which do no set forth the melting point or IR spectrum of the linezolid prepared.

The form of linezolid being used in the clinical trials to support the filing of the NDA is Form II.

### SUMMARY OF INVENTION

Disclosed is a (S)-N-[[3-[3-fluoro-4-(4-morpholinyl)phenyl]-2-oxo-5-oxazolidinyl]methyl]acetamide, crystal "Form II" with a powder X-ray diffraction spectrum of:

| d-Spacing (Á) | Two-Theta Angle (°) | Relative Intensity (%) |
|---|---|---|
| 12.44 | 7.10 | 2 |
| 9.26 | 9.54 | 9 |
| 6.37 | 13.88 | 6 |
| 6.22 | 14.23 | 24 |
| 5.48 | 16.18 | 3 |
| 5.28 | 16.79 | 100 |
| 5.01 | 17.69 | 2 |
| 4.57 | 19.41 | 4 |
| 4.50 | 19.69 | 2 |
| 4.45 | 19.93 | 6 |
| 4.11 | 21.61 | 15 |
| 3.97 | 22.39 | 23 |
| 3.89 | 22.84 | 4 |
| 3.78 | 23.52 | 7 |
| 3.68 | 24.16 | 1 |
| 3.52 | 25.28 | 13 |
| 3.34 | 26.66 | 1 |
| 3.30 | 27.01 | 3 |
| 3.21 | 27.77 | 1 |

Also disclosed is (S)-N-[[3-[3-fluoro-4-(4-morpholinyl)phenyl]-2-oxo-5-oxazolidinyl]methyl]acetamide, crystal "Form II" with an infrared (IR) spectrum as a mineral oil mull: 3364, 1748, 1675, 1537, 1517, 1445, 1410, 1401, 1358, 1329, 1287, 1274, 1253, 1237, 1221, 1145, 1130, 1123, 1116, 1078, 1066, 1049, 907, 852 and 758 cm⁻¹.

Further disclosed is a process to prepare (S)-N-[[3-[3-fluoro-4-(4-morpholinyl)phenyl]-2-oxo-5-oxazolidinyl]methyl]acetamide, crystal "Form II" which comprises:
(1) producing (S)-N-[[3-[3-fluoro-4-(4-morpholinyl)phenyl]-2-oxo-5-oxazolidinyl]methyl]acetamide in greater than 98% enantiomeric purity.
(2) mixing the greater than 98% enantiomerically pure (S)-N-[[3-[3-fluoro-4-(4-morpholinyl)phenyl]-2-oxo-5-oxazolidinyl]methyl]acetamide in a solvent or mixture of solvents at a temperature below a temperature of about 80° and
(3) separating the (S)-N-[[3-[3-fluoro-4-(4-morpholinyl)phenyl]-2-oxo-5-oxazolidinyl]methyl]acetamide crystal "Form II" from the solvent(s).

### DETAILED DESCRIPTION OF THE INVENTION

Linezolid, (S)-N-[[3-[3-fluoro-4-(4-morpholinyl)phenyl]-2-oxo-5-oxazolidinyl]methyl]acetamide, is a known pharmaceutically useful antibacterial agent, see US Patent 5,688,792 (EXAMPLE 5). Linezolid can be used orally or given by IV as a sterile solution.

When linezolid was originally produced, the crystal form was Form I. Form **II** differs from Form **I** in its **IR** spectrum, X-ray powder diffraction spectrum and melting point.

Once linezolid is synthesized, crystal Form II is prepared by starting with linezolid of high enantiomeric purity. It is preferred that the linezolid be more than 98% enantiomerically pure, it is more preferred that the linezolid be more than 99% pure and it is even more preferred that the linezolid be 99.5% pure. The linezolid of greater than 98% enantiomeric purity to be used to form crystal form II can either be in solution or be a solid. The linezolid starting material, solid or solution, is mixed with a solvent selected from the group consisting of:
water,
acetonitrile,
chloroform, methylene chloride, toluene,
R₁-OH where R₁ is C₁-C₆ alkyl,
R₁-CO-R₂ where R₂ is C₁-C₆ alkyl or phenyl substituted with 1 thru 3 R₁ where R₁ is as defined above, and where R₁ is as defined above,
R₁-CO-O-R₂ where R, is C₁-C₆ alkyl and R₁ is as defined above,
R₁-O-R₂ where R₁ is C₁-C₆ alkyl and R₁ is as defined above. It is preferred that the solvent be selected from the group consisting of water, ethyl acetate, methanol, ethanol, propanol, *i*-propanol, butanol, acetonitrile, acetone, methyl ethyl ketone, chloroform, methylene chloride, toluene, xylene, diethyl ether, or methyl-t-butyl ether. It is more preferred that the solvent be ethyl acetate, acetone, acetonitrile, propanol, or isopropanol. It is most preferred that the solvent be ethyl acetate.

The mixture of linezolid in the solvent is agitated at a temperature below 80° until crystals of Form II are formed and crystals of other solid forms, such as Form I, disappear. It is preferred to dissolve the linezolid in ethyl acetate at a temperature near the boiling point of the solvent. This mixture is cooled to a temperature of about 70°. The mixture may be seeded with crystals of Form II to facilitate crystallization. It is preferred that the solid product is cooled and agitated at a temperature between about 45° and about 60° until the solids consist only of Form II crystals. It is most preferred to maintain the slurry at a temperature of about 55°. It is preferred to mix the linezolid and solvent for at least 10 min, it is even more preferred to mix the linezolid and solvent for at least 20 min and it is most preferred to mix the linezolid and solvent for at least 30 min. The time and temperature will vary depending on the solvent selected. With ethyl acetate it is preferred to mix for not less that 60 minutes.

The crystalline slurry may be further cooled to improve yield, and the solid Form II product may be isolated. The mixture may be further cooled and agitated. Other measures which can be used to facilitate crystallization include, but are not limited to, cooling, concentration of the solution by evaporation or distillation, or through addition of other solvents.

The crystals are isolated by procedures known to those skilled in the art.

Crystal Form II is the most stable form below about 85°. It is preferred to use starting material with less than 0.2% of the R enantiomer of linezolid to minimize or eliminate the formation of a pseudoracemic solid solution of the two enantiomers which tends to crystallize as the Form I solid, even at temperatures below 85°.

It is well known to those skilled in the art that linezolid is useful as an antibacterial agent, see for example US Patent 5,688,792.

### DEFINITIONS AND CONVENTIONS

The definitions and explanations below are for the terms as used throughout this entire document including both the specification and the claims.

### DEFINITIONS

Linezolid refers to (S)-N-[[3-[3-fluoro-4-(4-morpholinyl)phenyl]-2-oxo-5-oxazolidinyl]methyl]acetamide the compound of formula:

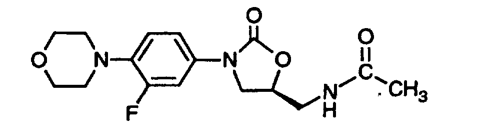

All temperatures are in degrees Centigrade.

IR refers to infrared spectroscopy.

Pharmaceutically acceptable refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

When solvent pairs are used, the ratios of solvents used are volume/volume (v/v).

When the solubility of a solid in a solvent is used the ratio of the solid to the solvent is weight/volume (wt/v).

The term C₁-C₆ alkyl means alkyl of 1 thru 6 carbon atoms and isomers thereof where such exist.

### EXAMPLES

Without further elaboration, it is believed that one skilled in the art can, using the preceding description, practice the present invention to its fullest extent. The following detailed examples describe how to prepare the various compounds and/or perform the various processes of the invention and are to be construed as merely illustrative, and not limitations of the preceding disclosure in any way whatsoever. Those skilled in the art will promptly recognize appropriate variations from the procedures both as to reactants and as to reaction conditions and techniques.

### EXAMPLE 1 Preparation of Crytal Form II of Linezolid

Linezolid with better than 99.8 % enantiomeric purity, less than 0.2% of the R enantiomer, (1.99 grams) is mixed with ethyl acetate (100 mL). The flask is stoppered and heated to 65° with constant stirring in a temperature controlled oil bath. The linezolid is completely dissolved and the mixture is stirred for an additional 10 minutes. The temperature is maintained at 55° in the flask and one neck of the flask is unstoppered to allow slow evaporation of the solvent. A gentle stream of nitrogen is blown across the open neck to aid in evaporation. Solids spontaneously precipitated from solution and the volume is reduced by about 25% of the initial volume. The flask is sealed and mixed for 90 minutes while maintaining the mixture at 55°. The mixture was then cooled to about 23° while being stirred. The solids are isolated by vacuum filtration using a sintered glass funnel to give linezolid in crystal form. Analysis by powder X-ray diffraction indicates that the solids are linezolid crystal Form II.

## Claims

1. (S)-N-[[3-[3-fluoro-4-(4-morpholinyl)phenyl]-2-oxo-5-oxazolidinyl] methyl] acetamide, crystal "Form II" with a powder X-ray diffraction spectrum of:
| d-Spacing (Á) | Two-Theta Angle (°) | Relative Intensity (%) |
|---|---|---|
| 12.44 | 7.10 | 2 |
| 9.26 | 9.54 | 9 |
| 6.37 | 13.88 | 6 |
| 6.22 | 14.23 | 24 |
| 5.48 | 16.18 | 3 |
| 5.28 | 16.79 | 100 |
| 5.01 | 17.69 | 2 |
| 4.57 | 19.41 | 4 |
| 4.50 | 19.69 | 2 |
| 4.45 | 19.93 | 6 |
| 4.11 | 21.61 | 15 |
| 3.97 | 22.39 | 23 |
| 3.89 | 22.84 | 4 |
| 3.78 | 23.52 | 7 |
| 3.68 | 24.16 | 1 |
| 3.52 | 25.28 | 13 |
| 3.34 | 26.66 | 1 |
| 3.30 | 27.01 | 3 |
| 3.21 | 27.77 | 1 |

2. (S)-N-[[3-[3-fluoro-4-(4-morpholinyl)phenyl]-2-oxo-5-oxazolidinyl]methyl] acetamide, crystal "Form II" with an infrared (IR) spectrum as a mineral oil mull: 3364, 1748, 1675, 1537, 1517, 1445, 1410, 1401, 1358, 1329, 1287, 1274, 1253, 1237, 1221, 1145, 1130, 1123, 1116, 1078, 1066, 1049, 907, 852 aand 758 cm⁻¹.

3. A process to prepare (S)-N-[[3-[3-fluoro-4-(4-morpholinyl)phenyl]-2-oxo-5-oxazolidinyl]methyl]acetamide, crystal "Form II" which comprises:
(1) producing (S)-N-[[3-[3-fluoro-4-(4-morpholinyl)phenyl]-2-oxo-5-oxazolidinyl]methyl]acetamide in greater than 98% enantiomeric purity,
(2) mixing the greater than 98% enantiomerically pure (S)-N-[[3-[3-fluoro-4-(4-morpholinyl)phenyl]-2-oxo-5-oxazolidinyl]methyl]acetamide in a solvent or mixture of solvents at a temperature below a temperature of about 80° and
(3) separating the (S)-N-[[3-[3-fluoro-4-(4-morpholinyl)phenyl]-2-oxo-5-oxazolidinyl]methyl]acetamide crystal "Form II" from the solvent(s).

4. A process according to claim 3 where the enantiomeric purity is greater than 99%.

5. A process according to claim 4 where the enantiomeric purity is greater than 99.5%.

6. A process according to claim 3 where the solvent is selected from the group consisting of
water,
acetonitrile,
chloroform, methylene chloride, toluene,
and compounds of the formulae
R₁-OH where R₁ is C₁-C₆ alkyl,
R₁-CO-R₂ where R₂ is C₁-C₆ alkyl or phenyl substituted with I thru 3 R₁, and where R₁ is as defined above,
R₁-CO-O-R₂ where R₁ and R₂ are as defined above,
R₁-O-R₂ where R₁ and R₂ are as defined above.

7. A process according to claim 6 where the solvent is selected from the group consisting of water, ethyl acetate, methanol, ethanol, propanol, *i*-propanol, butanol, acetonitrile, acetone, methyl ethyl ketone, chloroform, methylene chloride, toluene, xylene, diethyl ether, or methyl-t-butyl ether.

8. A process according to claim 6 where the solvent is selected from the group consisting of ethyl acetate, acetone, acetonitrile, propanol, or isopropanol.

9. A process according to claim 6 where the solvent is ethyl acetate.

10. A process according to claim 3 where the (S)-N-[[3-[3-fluoro-4-(4-morpholinyl)phenyl]-2-oxo-5-oxazolidinyl]methyl]acetamide is mixed for at least 10 min in the solvent or mixture of solvents.

11. A process according to claim 10 where the (S)-N-[[3-[3-fluoro-4-(4-morpholinyl)phenyl]-2-oxo-5-oxazolidinyl]methyl]acetamide is mixed for at least 20 min in the solvent or mixture of solvents.

12. A process according to claim 10 where the linezolid is mixed for at least 30 min in the solvent or mixture of solvents.

13. A process according to claim 3 where the temperature is less than about 75°.

14. A process according to claim 10 where the temperature is from about 45° to about 60°.

15. A process according to claim 3 where the (S)-N-[[3-[3-fluoro-4-(4-morpholinyl)phenyl]-2-oxo-5-oxazolidinyl]methyl]acetamide is isolated as a solid before mixing with a solvent or mixture of solvents.

16. A process according to claim 3 where the (S)-N-[[3-[3-fluoro-4-(4-morpholinyl)phenyl]-2-oxo-5-oxazolidinyl]methyl]acetamide is kept in solution before mixing with a solvent or mixture of solvents.

## Patentansprüche

1. Kristallines (S)-N-[[3-[3-Fluor-4-(4-morpholinyl)-phenyl]-2-oxo-5-oxazolidinyl]methyl]acetamid der "Form II" mit dem folgenden Pulverröntgenbeugungsspektrum:
| d-Abstand (Å) | 2θ-Winkel (°) | relative Intensität (%) |
|---|---|---|
| 12,44 | 7,10 | 2 |
| 9,26 | 9,54 | 9 |
| 6,37 | 13,88 | 6 |
| 6,22 | 14,23 | 24 |
| 5,48 | 16,18 | 3 |
| 5,28 | 16,79 | 100 |
| 5,01 | 17,69 | 2 |
| 4,57 | 19,41 | 4 |
| 4, 50 | 19,69 | 2 |
| 4,45 | 19,93 | 6 |
| 4,11 | 21,61 | 15 |
| 3,97 | 22,39 | 23 |
| 3,89 | 22,84 | 4 |
| 3,78 | 23,52 | 7 |
| 3,68 | 24,16 | 1 |
| 3,52 | 25,28 | 13 |
| 3,34 | 26,66 | 1 |
| 3,30 | 27,01 | 3 |
| 3,21 | 27,77 | 1 |

2. Kristallines (S)-N-[[3-[3-Fluor-4-(4-morpholinyl)-phenyl]-2-oxo-5-oxazolidinyl]methyl]acetamid der "Form II" mit dem folgenden Infrarot(IR)-Spektrum als Mineralölverreibung: 3364, 1748, 1675, 1537, 1517, 1445, 1410, 1401, 1358, 1329, 1287, 1274, 1253, 1237, 1221, 1145, 1130, 1123, 1116, 1078, 1066, 1049, 907, 852 und 758 cm⁻¹.

3. Verfahren zur Herstellung von kristallinem (S)-N-[[3-[3-Fluor-4-(4-morpholinyl)phenyl]-2-oxo-5-oxazolidinyl]-methyl]acetamid der "Form II", das umfasst:
(1) Herstellen von (S)-N-[[3-[3-Fluor-4-(4-morpholinyl)-phenyl]-2-oxo-5-oxazolidinyl]methyl]acetamid in einer Enantiomerenreinheit von größer als 98 %,
(2) Einmischen des zu mehr als 98 % enantiomerenreinen (S)-N-[[3-[3-Fluor-4-(4-morpholinyl)phenyl]-2-oxo-5-oxazolidinyl]methyl]acetamids in ein Lösemittel oder Lösemittelgemisch bei einer Temperatur unter einer Temperatur von etwa 80° und
(3) Abtrennen des kristallinen (S)-N-[[3-[3-Fluor-4-(4-morpholinyl)phenyl)-2-oxo-5-oxazolidinyl]methyl]-acetamids der "Form II" von dem Lösemittel bzw. den Lösemitteln.

4. Verfahren nach Anspruch 3, wobei die Enantiomerenreinheit mehr als 99 % beträgt.

5. Verfahren nach Anspruch 4, wobei die Enantiomerenreinheit mehr als 99,5 % beträgt.

6. Verfahren nach Anspruch 3, wobei das Lösemittel ausgewählt ist aus der Gruppe, die aus:
Wasser,
Acetonitril,
Chloroform, Methylenchlorid, Toluol,
und Verbindungen der Formel R₁-OH, worin R₁ C₁-C₆-Alkyl bedeutet,
R₁-CO-R₂, worin R₂ C₁-C₆-Alkyl oder Phenyl, das mit 1 bis 3 Resten R₁ substituiert ist, bedeutet und wobei R₁ wie im Vorhergehenden definiert ist,
R₁-CO-O-R₂, worin R₁ und R₂ wie im Vorhergehenden definiert sind,
R₁-O-R₂, worin R₁ und R₂ wie im Vorhergehenden definiert sind, besteht.

7. Verfahren nach Anspruch 6, wobei das Lösemittel aus der aus Wasser, Ethylacetat, Methanol, Ethanol, Propanol, Isopropanol, Butanol, Acetonitril, Aceton, Methylethylketon, Chloroform, Methylenchlorid, Toluol, Xylol, Diethylether oder Methyl-tert-butylether bestehenden Gruppe ausgewählt ist.

8. Verfahren nach Anspruch 6, wobei das Lösemittel aus der aus Ethylacetat, Aceton, Acetonitril, Propanol oder Isopropanol bestehenden Gruppe ausgewählt ist.

9. Verfahren nach Anspruch 6, wobei das Lösemittel Ethylacetat ist.

10. Verfahren nach Anspruch 3, wobei das (S)-N-[[3-[3-Fluor-4-(4-morpholinyl)phenyl]-2-oxo-5-oxazolidinyl]-methyl]acetamid mindestens 10 min in das Lösemittel oder Lösemittelgemisch eingemischt wird.

11. Verfahren nach Anspruch 10, wobei das (S)-N-[[3-[3-Fluor-4-(4-morpholinyl)phenyl]-2-oxo-5-oxazolidinyl]-methyl]acetamid mindestens 20 min in das Lösemittel oder Lösemittelgemisch eingemischt wird.

12. Verfahren nach Anspruch 10, wobei das Linezolid mindestens 30 min in das Lösemittel oder Lösemittelgemisch eingemischt wird.

13. Verfahren nach Anspruch 3, wobei die Temperatur weniger als etwa 75° beträgt.

14. Verfahren nach Anspruch 10, wobei die Temperatur von etwa 45° bis etwa 60° beträgt.

15. Verfahren nach Anspruch 3, wobei das (S)-N-[[3-[3-Fluor-4-(4-morpholinyl)phenyl]-2-oxo-5-oxazolidinyl]methyl]-acetamid als Feststoff isoliert wird, bevor es mit einem Lösemittel oder Lösemittelgemisch gemischt wird.

16. Verfahren nach Anspruch 3, wobei das (S)-N-[[3-[3-Fluor-4-(4-morpholinyl)phenyl]-2-oxo-5-oxazolidinyl]methyl]-acetamid in Lösung gehalten wird, bevor es mit einem Lösemittel oder Lösemittelgemisch gemischt wird.

## Revendications

1. "Forme cristalline II" de (S)-N-[[3-fluoro-4-(4-morpholinyl)phényl]-2-oxo-5-oxazolidinyl]méthyl]acétamide ayant le spectre de diffraction des rayons X sur poudre suivant :
| Distance d (Å) | Angle deux-thêta (°) | Intensité relative (%) |
|---|---|---|
| 12,44 | 7,10 | 2 |
| 9,26 | 9,54 | 9 |
| 6,37 | 13,88 | 6 |
| 6,22 | 14,23 | 24 |
| 5,48 | 16,18 | 3 |
| 5,28 | 16,79 | 100 |
| 5,01 | 17,69 | 2 |
| 4,57 | 19,41 | 4 |
| 4, 50 | 19,69 | 2 |
| 4,45 | 19,93 | 6 |
| 4,11 | 21,61 | 15 |
| 3,97 | 22,39 | 23 |
| 3,89 | .22,84 | 4 |
| 3,78 | 23,52 | 7 |
| 3,68 | 24,16 | 1 |
| 3,52 | 25,28 | 13 |
| 3,34 | 26,66 | 1 |
| 3,30 | 27,01 | 3 |
| 3,21 | 27,77 | 1 |

2. "Forme cristalline II" de (S) -N- [[3-[3-fluoro-4-(4-morpholinyl)phényl]-2-oxo-5-oxazolidinyl]méthyl]acétamide ayant un spectre infrarouge (IR), sous forme de pâte dans une huile minérale : 3364, 1748, 1675, 1537, 1517, 1445, 1410, 1401, 1358, 1329, 1287, 1274, 1253, 1237, 1221, 1145, 1130, 1123, 1116, 1078, 1066, 1049, 907, 852 et 758 cm⁻¹.

3. Procédé pour la préparation de la "forme cristalline II" de (S)-N-[[3- [3-fluoro-4-(4-morpholinyl)phényl]-2-oxo-5-oxazolidinyl]méthyl]acétamide, qui comprend les étapes consistant :
(1) à produire du (S)-N-[[3-[3-fluoro-4-(4-morpholinyl)phényl]-2-oxo-5-oxazolidinyl]méthyl]acétamide en une pureté d'énantiomère supérieure à 98 %,
(2) à mélanger le (S)-N-[[3-[3-fluoro-4-(4-morpholinyl)phényl]-2-oxo-5-oxazolidinyl]méthyl]acétamide énantiomériquement pur à 98 % dans un solvant ou mélange de solvants à une température inférieure à une température d'environ 80°C, et
(3) à séparer la "forme cristalline II" de (S)-N-[[3-[3-fluoro-4-(4-morpholinyl)phényl]-2-oxo-5-oxazolidinyl]méthyl]acétamide du ou des solvants.

4. Procédé suivant la revendication 3, dans lequel la pureté d'énantiomère est supérieure à 99 %.

5. Procédé suivant la revendication 4, dans lequel la pureté d'énantiomère est supérieure à 99,5 %.

6. Procédé suivant la revendication 3, dans lequel le solvant est choisi dans le groupe consistant en :
l'eau,
l'acétonitrile,
le chloroforme, le chlorure de méthylène, le toluène,
et des composés de formules :
R₁-OH dans laquelle R₁ représente un groupe alkyle en C₁ à C₆,
R₁-CO-R₂ dans laquelle R₂ représente un groupe alkyle en C₁ à C₆ ou phényle substitué avec 1 à 3 groupes R₁, les groupes R₁ répondant à la définition précitée,
R₁-CO-O-R₂ dans laquelle R₁ et R₂ répondent aux définitions précitées,
R₁-O-R₂ dans laquelle R₁ et R₂ répondent aux définitions précitées.

7. Procédé suivant la revendication 6, dans lequel le solvant est choisi dans le groupe consistant en eau, acétate d'éthyle, méthanol, éthanol, propanol, isopropanol, butanol, acétonitrile, acétone, méthyléthylcétone, chloroforme, chlorure de méthylène, toluène, xylène, éther diéthylique et éther méthylique de tertiobutyle.

8. Procédé suivant la revendication 6, dans lequel le solvant est choisi dans le groupe consistant en acétate d'éthyle, acétone, acétonitrile, propanol et isopropanol.

9. Procédé suivant la revendication 6, dans lequel le solvant est l'acétate d'éthyle.

10. Procédé suivant la revendication 3, dans lequel le (S)-N-[[3-[3-fluoro-4-(4-morpholinyl)phényl]-2-oxo-5-oxazolidinyl]méthyl]acétamide est mélangé pendant au moins 10 minutes dans le solvant ou le mélange de solvants.

11. Procédé suivant la revendication 10, dans lequel le (S)-N- [ [3- [3-fluoro-4- (4-morpholinyl)phényl] -2-oxo-5-oxazolidinyl]méthyl]acétamide est mélangé pendant au moins 20 minutes dans le solvant ou mélange de solvants.

12. Procédé suivant la revendication 10, dans lequel le linézolide est mélangé pendant au moins 30 minutes dans le solvant ou mélange de solvants.

13. Procédé suivant la revendication 3, dans lequel la température est inférieure à environ 75°.

14. Procédé suivant la revendication 10, dans lequel la température est comprise dans l'intervalle d'environ 45° à environ 60°.

15. Procédé suivant la revendication 3, dans lequel le (S)-N-[[3-[3-fluoro-4-(4-morpholinyl)phényl]-2-oxo-5-oxazolidinyl]méthyl]acétamide est isolé sous forme d'une substance solide avant d'être mélangé à un solvant ou mélange de solvants.

16. Procédé suivant la revendication 3, dans lequel le (S)-N-[[3-[3-fluoro-4-(4-morpholinyl)phényl]-2-oxo-5-oxazolidinyl]méthyl]acétamide est maintenu en solution avant d'être mélangé à un solvant ou mélange de solvants.
